# EUROPEAN PATENT APPLICATION

(11) **EP 0 943 342 A2**
(43) Date of publication of application: **22.09.1999**
(21) Application number: 99301921.5
(22) Date of filing: 12.03.1999
(51) Int. Cl.: A61L 2/12

(54) **Method of sterilizing medical instruments**

(30) Priority: 16.03.1998 US 39437
(71) Applicant: Fort James Operating Company, Deerfield, IL 60015 (US)
(72) Inventor: Habeger, Charles C., Jr., Smyrna, GA 30080 (US)
(74) Representative: Cropp, John Anthony David

(57) **Abstract**

When a medical instrument is sterilized by subjecting it to microwave radiation in a container comprising a susceptor that converts the radiation into heat, the tendency of the instrument to arc can be reduced by vacuum sealing the container. When the container is a pouch made from a flexible material, the vacuum sealing causes the material to intimately contact the medical instrument, thereby increasing the rate at which heat is transferred from the material to the instrument.

## Description

This invention relates to an improvement in the method of sterilizing medical instruments by subjecting the instruments to microwave radiation in a container having a susceptor.

### Background of the Invention

U.S. Patents 5,552,112, 5,599,499, 5,607,612 and 5,645,748, which are incorporated herein by reference, disclose that medical instruments, such as dental tools, can be sterilized by placing the instruments in a container comprising a microwave susceptor, and then exposing the container to microwave radiation. The susceptor converts the radiation to heat that is imparted to the instruments. However, one problem with this method, as recognized in the patents, is that the instruments tend to arc. This invention provides an improved method to prevent arcing and to increase the rate at which the instruments are heated.

### Summary of the Invention

The improved method of this invention comprises placing a medical instrument in a container comprising a microwave susceptor, substantially evacuating the atmosphere in the container, hermetically sealing the container, and subjecting the container to microwave radiation in an amount that is sufficient to sterilize the instrument.

The atmosphere in the container, which is normally ambient air, may be evacuated by any well known method for vacuum packaging an object. Reducing the pressure of the air or other gas in the container decreases the tendency of the instrument to arc.

The container may be either rigid or flexible, but preferably is a pouch made from a flexible material. When the air in the pouch is evacuated, the interior surface of the pouch intimately envelops the medical instrument, thereby providing better transfer of heat from the susceptor to the instrument.

The method of this invention is preferably carried out at a frequency of 5.80 GHz. Most microwave ovens operate at a frequency of 2.45 GHz, but the frequency of 5.80 GHz has also been allocated for commercial microwave ovens. Increasing the frequency of the microwave radiation reduces the tendency of the instrument to arc.

The microwave susceptor is preferably a metallized plastic film. The metallized film is typically laminated to a dimensionally stable substrate, such as paper, using a conventional adhesive, but when the plastic film itself is dimensionally stable at the temperatures generated by the microwave radiation, it is not necessary to laminate the metallized film to a substrate.

The invention will now be described in greater detail with reference to preferred embodiments and with the aid of the accompanying drawings in which
Figure 1 is a plan view of a medical instrument vacuum sealed in a container in accordance with this invention; and
Figure 2 is a sectional view of the instrument and container taken along line 2-2. The thicknesses of the layers are exaggerated to illustrate the invention.

### Description of the Preferred Embodiment

In a preferred embodiment of this invention, the container is a pouch 10 that is made by folding over a sheet of flexible material and sealing it along two side edges 12, 14 to form a pouch having a top end 16 that is open initially to receive a medical instrument, such as a dental explorer 18. Two or more instruments may be inserted if desired, provided they are spaced apart sufficiently to avoid arcing. After the instrument 18 is inserted into the pouch 10, the atmosphere (ambient air) in the pouch is substantially evacuated, such as by using a vacuum pump, in accordance with conventional methods. The top end 16 of the pouch 10 is then hermetically sealed in accordance with conventional methods, such as by heat sealing a layer of plastic film to itself or by applying an adhesive to the interior surface of the pouch at the marginal edges of the top end 16.

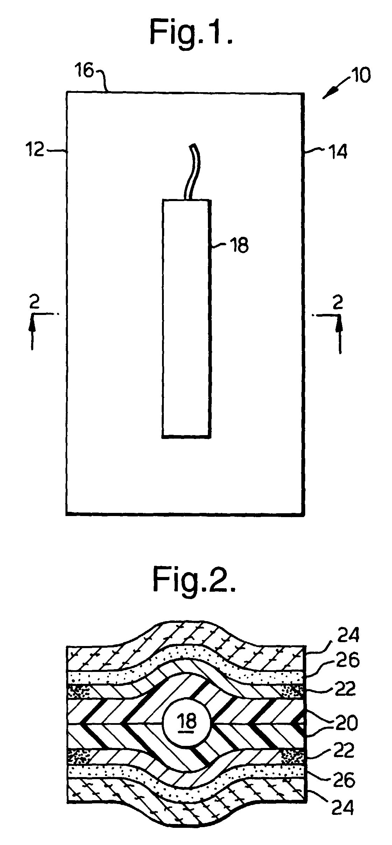

As shown in Figure 2, the pouch preferably comprises a layer of plastic film 20 on which is deposited, such as by vacuum deposition, a very thin layer of metal 22, such as aluminum, having a thickness such that it absorbs microwave energy and converts it into heat. The film 20 is preferably made from a polyester, such as polyethylene terephthalate. The metallized plastic film is laminated to a layer of a dimensionally stable substrate 24, such as paper or cellophane, by means of an adhesive 26. The metal at the top and side marginal edges of the pouch is preferably inactivated, as described in US. Patent 4,865,921, to avoid heating that might disrupt the seal at the edges. Alternatively, the metallized plastic film can be applied to the substrate 24 as a patch that contacts the instrument(s) in the pouch, but does not extend to the sealed edges of the pouch. Material suitable for forming pouches for use herein are disclosed in U.S. Patent 4,890,439, which is incorporated herein by reference, and are available commercially under the trademark MICROFLEX® Q.

In another embodiment, the pouch can be made from two different, overlying sheets of material sealed at their marginal edges, with one sheet being a layer of metallized plastic laminated to a dimensionally stable substrate, as described above, and the other sheet being a clear plastic film that is heat stable at the temperatures generated in the microwave oven. The clear plastic film provides a window to enable the user to see the instruments in the pouch.

When the vacuum sealed pouch 10 containing the dental explorer 18 is heated in a microwave oven, preferably operated at a frequency of 5.80 GHz, less arcing is observed compared to heating the explorer in a pouch wherein the atmosphere has not been evacuated, and the explorer is heated at a faster rate.

## Claims

1. A method of sterilizing a medical instrument by placing the instrument in a container comprising a microwave susceptor, hermetically sealing the container, and subjecting the container to microwave radiation to sterilize the instrument, wherein the atmosphere in the container is substantially evacuated before the container is hermetically sealed.

2. A method as claimed in Claim 1 wherein the container is a pouch made from a flexible material and the pouch intimately envelopes the instrument when the atmosphere in the container is evacuated.

3. A method as claimed in Claim 1 or Claim 2 wherein the susceptor comprises a metallized plastic film.

4. A method as claimed in Claim 3 wherein the plastic film is laminated to a dimensionally stable substrate.

5. A method as claimed in Claim 4 wherein the substrate is paper or cellophane.

6. A method as claimed in Claim 3 wherein the plastic film itself is dimensionally stable.

7. A method as claimed in any one of the preceding claims wherein the microwave radiation has a frequency of about 5.8 GHz.
